# EUROPÄISCHE PATENTSCHRIFT

(11) **EP 1 075 482 B1**
(45) Veröffentlichungstag und Bekanntmachung des Hinweises auf die Patenterteilung: **03.04.2002**
(21) Anmeldenummer: 99922156.7
(22) Anmeldetag: 03.05.1999
(51) Int. Cl.: C07F 15/00, C08F 4/80, B01J 31/00, C07C 6/04

(54) **KATIONISCHE RUTHENIUMKOMPLEXE, IHRE HERSTELLUNG UND IHRE VERWENDUNG**
CATIONIC RUTHENIUM COMPLEXES, THEIR PRODUCTION AND THEIR USE
COMPLEXES CATIONIQUES DE RUTHENIUM, LEUR PREPARATION ET LEUR UTILISATION

(30) Priorität: 08.05.1998 DE 19820652
(43) Veröffentlichungstag der Anmeldung: 14.02.2001
(73) Patentinhaber: BASF AKTIENGESELLSCHAFT, 67056 Ludwigshafen (DE)
(72) Erfinder: SCHWAB, Peter, D-67098 Bad Dürkheim (DE); SCHULZ, Michael, D-67067 Ludwigshafen (DE); WOLF, Justin, D-97074 Würzburg (DE); STUER, Wolfram, D-97080 Würzburg (DE); WERNER, Helmut, D-97074 Würzburg (DE)
(74) Vertreter: Isenbruck, Günter
(86) Internationale Anmeldenummer: EP9902992
(87) Internationale Veröffentlichungsnummer: WO9958538

(56) Entgegenhaltungen:
- WO-A-97/06185
- STUER, WOLFRAM ET AL: "Carbynehydridoruthenium complexes as catalysts for the selective, ring-opening metathesis of cyclopentene with methyl acrylate" ANGEW. CHEM., INT. ED. (1999), VOLUME DATE 1998, 37(24), 3421-3423 ,1999, XP002108794

## Beschreibung

Die Erfindung betrifft kationische Rutheniumkomplexe, die beispielsweise als Katalysatoren in Metathesereaktionen eingesetzt werden können, und Verfahren zu ihrer Herstellung.

Bei der Olefinmetathese (Disproportionierung) findet in ihrer einfachsten Form eine reversible, metallkatalysierte Umalkylidenierung von Olefinen durch Bruch und Neuformierung von Kohlenstoff-Kohlenstoff-Doppelbindungen statt. Im Fall der Metathese acyclischer Olefine unterscheidet man beispielsweise zwischen einer Selbstmetathese, bei der ein Olefin in ein Gemisch zweier Olefine unterschiedlicher molarer Massen übergeht (beispielsweise Umsetzung von Propen zu Ethen und 2-Buten), und der Kreuz- oder Co-Metathese, die eine Umsetzung zweier unterschiedlicher Olefine beschreibt (beispielsweise von Propen mit 1-Buten zu Ethen und 2-Penten). Zu den weiteren Anwendungsgebieten der Olefinmetathese zählen Synthesen ungesättigter Polymere durch ringöffnende Metathesepolymerisation (ROMP) von cyclischen Olefinen und die acyclische Dien-Metathesepolymerisation (ADMET) von α,ω-Dienen. Neuere Anwendungen betreffen die selektive Ringöffnung von cyclischen Olefinen mit acyclischen Olefinen, sowie Ringschlußreaktionen (RCM), mit denen - vorzugsweise aus α, ω-Dienen - ungesättigte Ringe verschiedener Ringgröße hergestellt werden können.

Als Katalysatoren für Metathesereaktionen eignen sich prinzipiell homogene und heterogene Übergangsmetallverbindungen.

Heterogene Katalysatoren, beispielsweise Molybdän-, Wolfram- oder Rheniumoxide auf anorganischen oxidischen Trägern, zeichnen sich bei Reaktionen von nichtfunktionalisierten Olefinen durch hohe Aktivität und Regenerierbarkeit aus, müssen allerdings beim Einsatz funktionalisierter Olefine, wie Methyloleat, zur Aktivitätssteigerung oft mit einem Alkylierungsmittel vorbehandelt werden. Olefine mit protischen funktionellen Gruppen (wie Hydroxylgruppen, Carboxylgruppen oder Aminogruppen) führen zur spontanen Desaktivierung des Heterogenkontaktes.

In den letzten Jahren wurden vermehrt Anstrengungen unternommen, in protischem Medium und an Luftsauerstoff stabile Homogenkatalysatoren herzustellen. Besonderes Interesse haben dabei spezielle Ruthenium-Alkylidenverbindungen gefunden. Derartige Komplexe und Verfahren zu ihrer Herstellung sind bekannt.

In der WO 93/20111 sind Ruthenium- und Osmiummetallcarbenkomplexe für die Olefinmetathesepolymerisation beschrieben. Die Komplexe weisen die Struktur RuX₂(=CH-CH=CR₂)L₂ auf. Als Liganden L kommen Triphenylphosphan und substituiertes Triphenylphosphan zum Einsatz. Die Herstellung erfolgt beispielsweise durch Umsetzung von RuCl₂(PPh₃)₃ mit geeigneten disubstituierten Cyclopropenen als Carben-Vorläufern. Die Synthese von Cyclopropen-Derivaten ist jedoch mehrstufig und wirtschaftlich wenig interessant.

Ähnliche Umsetzungen sind in WO 96/04289 beschrieben. Zudem sind Verfahren zur Olefinmetathesepolymerisation angegeben.

Eine Verwendung derartiger Katalysatoren für die Peroxidvernetzung von ROMP-Polymeren ist in WO 97/03096 beschrieben.

In der WO 97/06185 sind ebenfalls Metathesekatalysatoren beschrieben, die auf Rutheniummetallcarbenkomplexen beruhen. Sie können neben dem vorstehend beschriebenen Verfahren auch durch Umsetzung von RuCl₂(PPh₃)₃ mit Diazoalkanen hergestellt werden. Der Umgang mit Diazoalkanen stellt jedoch, besonders bei der Durchführung des Verfahrens in technischem Maßstab, ein Sicherheitsrisiko dar.

Zudem müssen die eingesetzten metallorganischen Startmaterialien der Formel RuCl₂(PPh₃)₃ unter Einsatz eines großen Überschußes an Triphenylphosphan aus RuCl₃ 3 H₂O hergestellt werden. Bei der Katalysatorsynthese an sich gehen dann nach Ligandenaustausch nochmals PPh₃-Liganden verloren. Die eingesetzten Carben-Vorläufer erfordern mehrstufige Synthesen und sind nicht unbegrenzt haltbar.

In Organometallics 1996, 15, 1960 bis 1962 ist ein Verfahren zur Herstellung von Rutheniumkomplexen beschrieben, bei dem polymeres [RuCl₂(Cyclooctadien)]ₓ in i-Propanol in Gegenwart von Phosphan mit Wasserstoff umgesetzt wird. Dadurch entfällt die Notwendigkeit des Phosphanaustausches. Es wird ein undefiniertes Gemisch von Produkten erhalten Zudem sind ausgehend von einem polymeren Startmaterial lange Reaktionszeiten erforderlich. Das im metallorganischen Edukt enthaltene Cyclooctadien trägt nicht zur Reaktion bei und geht verloren.

In J. Chem. Soc. Commun. 1997, 1733 bis 1734 ist eine Synthese des Methylenkomplexes RuCl₂(=CH₂)(PCy₃)₂ beschrieben, die von Dichlormethan und dem Ruthenium-Polyhydrid-Komplex RuH₂(H₂)₂(PCy₃)₂ ausgeht. Der Ruthenium-Polyhydrid-Komplex ist jedoch schwer zugänglich. Zudem sind lange Reaktionszeiten erforderlich.

Die vorstehenden Ruthenium(II)-Alkylidenkomplexe sind ebenso wie alle anderen bekannten Metathesekatalysatoren elektronenreicher Übergangsmetalle als Katalysatoren für Metathesereaktionen von elektronenarmen Olefinen wie Acrylsäure oder Derivaten davon nicht oder nur wenig geeignet.

Katalysatorsysteme auf Basis von Molybdän und Wolfram eignen sich nur sehr bedingt zu Metathesereaktionen der funktionalisierten Olefine. Die aktivsten Katalysatoren im Bereich elektronenarmer Übergangsmetalle, wie z.B. in EP-A-0 218 138 beschriebene Systeme des Typs (RO)₂M(NR)(=CHR') (M = Mo, W), haben außer einer nur geringen Aktivität gegenüber derartigen Substraten zudem den Nachteil einer extrem hohen Empfindlichkeit gegenüber Verunreinigungen im Feed und sind ferner aufgrund sehr hoher Herstellkosten wirtschaftlich nicht von Interesse. Crowe beschreibt in J. Am. Chem. Soc. 1995, 117, 5162 bis 5163 den Einsatz dieser Katalysatoren für mechanistische Studien zu Kreuzmetathesereaktionen von Acrylnitril (H₂C=CHCN) mit H₂C=CHR (R = elektronenschiebender Rest) zu NCHC=CHR und H₂C=CH_{2'} die selbst bei hoher Katalysatorkonzentration nur in mäßigen Umsätzen ablaufen. US 5,621,047 beschreibt die ringöffnende Kreuzmetathese von Cyclooctadien mit Methylmethacrylat zu Oligomeren mit carboxylischen Endgruppen unter Einsatz von WCl₆/SnMe₄.

Kostengünstige und gegenüber Feed-Verunreinigungen beziehungsweise Luftsauerstoff stabile Katalysatorsysteme für Metathesereaktionen, an denen elektronenarme Olefine beteiligt sind, sind bislang nicht bekannt.

Es stellte sich somit die Aufgabe, ein Katalysatorsystem für Metathesereaktionen elektronenarmer Olefine zu entwickeln, um großtechnische Grundprodukte, wie Acrylsäure und Derivate davon, Acrylnitril, Vinylchlorid, Vinylsulfon etc. für Metathesereaktionen zugänglich zu machen. Neben einer hohen Aktivität sollten eine hohe Stabilität gegenüber Feed-Verunreinigungen und Luftsauerstoff sowie eine hohe Standzeit sowie ein kostengünstiger und unkomplizierter Zugang aus verfügbaren Rohstoffen verwirklicht werden.

Diese Aufgabe wurde erfindungsgemäß gelöst durch Katalysatorsysteme, welche als Aktivkomponenten kationische Rutheniumkomplexe der allgemeinen Formeln A (kationische Carbinkomplexe) oder B (kationische Carbenkomplexe) oder diese enthaltende Gemische 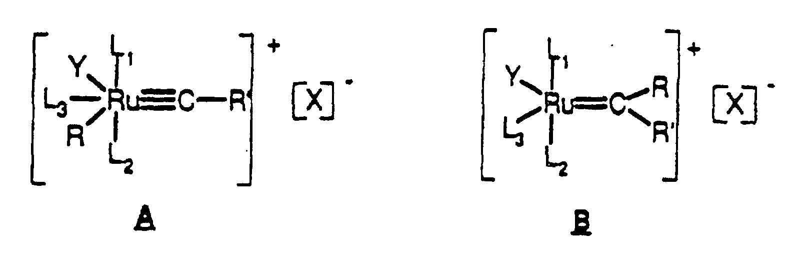 aufweisen, wobei B durch einen weiteren Liganden L₄ stabilisiert sein kann. In den Strukturen A und B bedeuten
- X⁻: ein nicht- oder schwach an das Metallzentrum koordinierendes Anion, wie Komplexanionen aus der III. bis VII. Hauptgruppe des Periodensystems der Elemente, beispielsweise BR"₄⁻(R"=F, Phenylreste, die durch ein oder mehrere Fluoratome oder mehrfach oder perfluorierte C₁₋₆-Alkylreste substituiert sein können, wie C₆H₅₋ₙFₙ mit n = 1 bis 5), PF₆⁻, AsF₆⁻, SbF₆⁻, ClO₄⁻, CF₃SO₃⁻ oder FSO₃⁻,
- Y: einen ein- oder mehrzähnigen anionischen Liganden
- R und R': jeweils unabhängig voneinander Wasserstoff oder einen, gegebenenfalls substituierten, C₁₋₂₀-Alkyl-, C₆₋₂₀-Aryl- oder C₇₋₂₀-Alkylaryl oder Aralkylrest,
L₁, L₂, L₃ und L₄ unabhängig voneinander neutrale Elektonendonor-Liganden, vorzugsweise Stickstoff-Donoren, wie Amine und Pyridine, Phosphane, Arsane, Stibane mit mindestens zwei sterisch anspruchsvollen Resten, wie i-Propyl, t-Butyl, Cyclopentyl, Cyclohexyl, Menthyl oder ähnliches, aber auch π-koordinierte Olefine oder Lösemittelmoleküle.

Vorzugsweise haben die Reste folgende Bedeutungen:
- X⁻: BR4"₄₋ mit R" = F oder C₆H₃ (m - CF₃)₂,
- Y: Halogen, vorzugsweise Chlor, oder OR mit R = C₁₋₆-Alkyl, C₆₋₁₂-Aryl, vorzugsweise Phenolat,
- R: H
- R': C₁₋₆-Alkyl, C₆₋₁₂-Aryl, C₇₋₂₀-Aralkyl, vorzugsweise Methyl oder Benzyl,
- L₁, L₂: Phosphane mit mindestens zwei sterisch anspruchsvollen Resten,
- L₃, L₄: cyclische oder acyclische Ether oder tertiäre Amine wie NMe₂Phenyl, NMe₃, NEt₃.

Die Synthese der Aktivkomponenten A und/oder B beziehungsweise von Gemischen, die diese Aktivkomponenten enthalten, kann ausgehend von zahlreichen metallorganischen Ausgangsstoffen erfolgen, beispielsweise
- durch Umsetzung von Hydrido(vinyliden)komplexen des Typs RuY(H)(=C=CHR)L₁L₂, deren Synthese durch Umsetzung von RuClH(H₂)L₁L₂ mit terminalen Alkinen HC≡CR erfolgen kann, mit
   R⁺X⁻, wobei X⁻ ein nicht- oder schwach koordinierendes Anion darstellt.
   RuClH(H₂)L₂ kann hierbei gemäß Literaturangaben beispielsweise aus dem polymeren Ruthenium-Precursor [RuCl₂(COD)]ₓ (COD = Cyclooctadien) in i-Propanol in Gegenwart von L unter Wasserstoffatmosphäre (Werner et al., Organometallics 1996, 15, 1960 bis 1962) oder ausgehend vom selben Startmaterial in sec-Butanol in Gegenwart von L und tert. Aminen (NEt₃) unter Wasserstoffatmosphäre (Grubbs et al., Organometallics 1997, 16, 3867 bis 3869) hergestellt werden. RuClH(H₂)L₂ ist ferner ausgehend von RuCl₃·H₂O in THF durch Umsetzung mit L in Gegenwart von aktiviertem Magnesium unter Wasserstoffatmosphäre (BASF AG, prioritätsältere, nicht vorveröffentlichte DE-A-198 00 934) zugänglich und wird vorzugsweise in situ mit 1-Alkinen zu den entsprechenden Hydrido(chloro)vinylidenkomplexen RuClH(=C=CHR)L₂ umgesetzt. Letztere können isoliert werden oder reagieren in situ mit H⁺X⁻ (X⁻ = nichtkoordinierendes Anion) zu den erfindungsgemäßen Aktivkomponenten A und/oder B.
- Durch Umsetzung von Verbindungen des Typs RuYY'(=CHR)L₁L₂ (wobei Y = Y' sein kann) mit R⁺X⁻, wobei X' ein nicht- oder schwach koordiniertes Anion darstellt. Gemischt-anionische Alkylidenkomplexe RuXY (=CHCH₂R)L₂ können gemäß DE-A-198 00 934 ausgehend von RuXH(=C=CHR)L₂ hergestellt werden. R⁺X kann eine saure Verbindung H⁺X⁻ oder LH⁺X⁻ darstellen, wobei X⁻ ein nicht- oder schwach koordinierendes Anion oder Komplexanion darstellt und L ein Zweielektronendonor-Ligand ist
- R⁺X⁻ kann z.B. CF₃SO₃H, F₃CCO₂H, HCIO₄, HBF₄, HPF₆, HSbF₆ oder [PhNMe₂H] [B(C₆F₅)₄] bedeuten.
- Durch Umsetzung von Verbindungen des Typs RuYY'(=CHR)L₁L₂ mit anionen-abstahierenden Metallsalzen M⁺X⁻ beziehungsweise Lewissäuren, wie BF₃ oder AlCl₃ in Gegenwart eines Liganden L₃, wobei X⁻ ein nicht oder nur schwach koordinierendes Anion darstellt und die anionischen Liganden Y und Y' gleich oder voneinander verscheiden sein können. Dabei können als anionen- abstrahierende Metallsalze M⁺X⁻ solche Salze eingesetzt werden, bei denen M⁺ mit den zu abstrahierenden Anionen ein schwerlösliches neues Metallsalz bildet und X⁻ ein nicht- oder schwach koordinierendes Anion darstellt. MX kann beispielsweise AgPF₄, AgB(C₅F₅)₄, AgPF₆ oder AgSbF₆ sein.

R⁺X⁻, M⁺X⁻ und die entsprechenden Lewissäuren werden vorzugsweise, bezogen auf das metallorganische Edukt, im Molverhältnis 1 : 10 bis 1000 : 1 eingesetzt.

Umsetzungen zu den Aktivkomponenten A und/oder B werden vorzugsweise in organischen Lösungsmitteln unter Inertgasatmosphäre durchgeführt, vorzugsweise in solchen Lösungsmitteln, die ein ungesättigtes Metallzentrum durch Koordination stabilisieren können, wie aliphatische oder cyclische Ether wie Dioxan oder THF, Amine, DMSO, Nitrile, Phosphane, Arsane, Stibane, Wasser, Olefine oder sonstige Zweielektronendonoren. Bevorzugt wird die Reaktion in THF bei Temperaturen von -100 bis +100°C, bevorzugt -80 bis -40°C und Drücken von 1 mbar bis 100 bar, bevorzugt bei 0,5 bis 5 bar durchgeführt.

Die Umsetzung kann mit einem oder mehr Moläquivalenten R⁺X⁻ erfolgen. Bei Einsatz von überschüssigem R⁺X⁻ gebildetes L₁₋₃RX beeinträchtigt die Reaktion nicht. Die hierbei erhaltenen, die Aktivkomponenten A und/oder B enthaltenden Zusammensetzungen können in situ als hochaktives Metathese.Katalysatorsystem eingesetzt werden oder bei tiefen Temperaturen unter Inertgasatmosphäre gelagert werden.

Gegebenenfalls werden die Aktivkomponenten A oder B in isolierter Form eingesetzt.

In der Regel ist die Reaktion nach 1 s bis 10 h, vorzugsweise nach 3 s bis 1 h beendet. Als Reaktionsgefäße eignen sich allgemein Glas- oder Stahlbehälter, welche gegebenenfalls mit Keramik ausgekleidet werden.

Die Komponenten A und/oder B beziehungsweise Mischungen, die diese Komponenten enthalten, sind hochaktive Metathesekatalysatoren für lineare und cyclische Olefine.

Die Katalysatorsysteme können unter anderem eingesetzt werden für
- Selbstmetathese eines Olefins oder Kreuzmetathese zweier oder mehrerer Olefine
- ringöffnende Metathesepolymerisation (ROMP) von cyclischen Olefinen
- selektive Ringöffnung von cyclischen Olefinen mit acyclischen Olefinen
- Acyclische Dien-Metathesepolymerisation (ADMET)
- Ringschlußmetathese (RCM)
- und zahlreiche Metathesevarianten.

Im Gegensatz zu RuCl₂(=CHR)L₂ können bei Anwendungen dieser Katalysatorsysteme erstmals elektronenarme Olefine des Typs R^{a}R^{b}C=CR^{c}Z, wobei R^{a}, R^{b}, R^{c} unabhängig voneinander Wasserstoff, einen C₁₋₁₂-Alkyl- oder C₆₋₁₂-Arylrest und Z einen elektronenziehenden Rest wie CO₂R^{d}, CONH₂, COX, CN mit X Halogen und R^{d} Wasserstoff, C₁₋₁₂-Alkylrest oder C₆₋₁₂-Arylrest darstellt, effizient und bereits bei milden Reaktionsbedingungen mit sehr hoher Aktivität umgesetzt werden. Als Beispiele hierfür seien genannt Acrylsäure und Derivate, Fumar- und Maleinsäureester, Maleinsäureanhydrid, Vinylchlorid, Methylvinylketon und andere.

Die Erfindung wird nachstehend durch Beispiele näher erläutert.

### Beispiele

### Beispiel 1: Herstellung von RuHCl(H₂)(PCy₃)₂:

Eine braune Suspension von 300 mg (1.07 mmol) [RuCl₂(C₈H₁₂)]ₙ und 982 mg (3.50 mmol) Tricyclohexylphosphan in 15 ml 2-Butanol wird unter H₂-Atmosphäre (1 bar) 2,5 h bei 110°C gerührt, wobei sich eine orangenrote Lösung bildet und ein orangeroter Feststoff ausfällt. Nach Abkühlen auf Raumtemperatur wird der Feststoff abfiltriert, in der Kälte dreimal mit jeweils 5 ml Aceton gewaschen und im Vakuum getrocknet.
Ausbeute: 559 mg (75%).

### Beispiel 2: Herstellung von RuHCl(=C=CH₂)(PCy₃)₂:

In eine auf -78°C gekühlte Lösung von 102 mg (0.15 mmol) RuHCl(H₂)(PCy₃)₂ aus Beispiel 1 in CH₂Cl₂ wird unter Rühren für ca. 30 s Acetylen eingeleitet. Das Lösungsmittel wird im Vakuum entfernt, der braune Feststoff mit 5 ml kaltem Pentan gewaschen und im Vakuum getrocknet.
Ausbeute: 99 mg (94%).

### Beispiel 3: Umsetzung von RuHCl(=C=CH₂)(PCy₃)₂ mit [PhNMe₂H]-[B(C₆F₅)₄] Darstellung von [RuClH(≡CMe)(NMe₂Ph)(PCy₃)₂][B(C₆F₅)₄] 1

72 mg (0,10 mmol) RuHCl(=C=CH₂)PCy₃)₂ aus Beispiel 2 werden als Feststoff mit 80 mg (0.10 mmol) [PhNMe₂H][B(C₆F₅)₄] vermischt und nach Abkühlen auf -80°C mit 10 ml CH₂Cl₂ versetzt. Hierbei verfärbt sich die Reaktionsmischung spontan gelb. Der hierbei gebildete Komplex 1 wurde spektroskopisch eindeutig identifiziert (Angaben in ppm). Laut NMR verläuft die Umsetzung quantitativ.
¹H-NMR (200 MHz, CD₂Cl₂, -60°C): δ = 7.32-6.97 (m, 5H, Ph-H), 3.0-1.2 (m, 75 H, PCy₃- und Me-H), - 6.33 (+, ²J_{PH} = 14.5 Hz, 1H, RuH);
¹⁹F-NMR (376.4 MHz, CD₂Cl₂, -70°C): δ = 133.3 (s), -162.5 (s), -166.4 (s);
³¹P-NMR (162.0 MHz, CD₂Cl₂, -70°C): δ = 56.6 (s, PCy₃);
¹³C-NMR (100.6 MHz, CD₂Cl₂, -70°C): δ = 311.9 (s breit, Ru≡C-Me), 148.6, 146.2, 138.9, 137.0, 134.5 und 129.1 (je s, Ph-C bzw. C_{Ipso} von C₆F₅), 136.4 (t, C_{Ipso} von C₆F₅ bzw. Ph-C), 123.2, 118.1 und 113.4 (je m, C₆F₅), 41.5 (s breit, NMe), 40.2 (s, Ru≡C-Me), 34.0 (vt, N = 22.5 Hz, Cₐ von PCy₃), 30.6, 28.9, 26.7, 26.4 und 25.3 (je s, PCy₃).

### Beispiel 4: Umsetzung von RuHCl(=C=CH₂)(PCy₃)₂ mit HBF₄ in Et₂O Darstellung von [RuClH(≡C-Me)(OEt₂)(PCy₃)₂][BF₄] 2

102 mg (0.14 mmol) RuHCl(=C=CH₂)(PCy₃)₂ aus Beispiel 2 werden in einem Gemisch von 5 ml CH₂Cl₂ und 5 ml Et₂O gelöst. Die rotbraune Lösung wird bei -80°C mit einem Überschuß einer Lösung von HBF₄ in Et₂O versetzt (ca. 0.1 ml einer 1.6 M Lösung von HBF₄ in Et₂O). Die braune Lösung wird auf ca. 0°C erwärmt und das Solvens im Vakuum enternt. Der braune Rückstand wird mit 5 ml Et₂O versetzt und einige Minuten gerührt, wobei sich ein gelber Feststoff bildet. Die überstehende braune Lösung wird verworfen und der gelbe Feststoff 2 nochmals mit 5 ml Et₂O gewaschen.
Ausbeute: 90 mg (72%).
Leitfähigkeit (CH₂Cl₂, 20°C): Λ = 47 cm² Ω⁻¹ mol⁻¹
¹H-NMR (400.1 MHz, CD₂Cl₂, 28°C): δ = 4.77 (q, J_{HH} = 7,2 Hz, 4 H, OCH₂CH₃), 2.52 - 1.30 (m, 75 H, CH₃, PCy₃), -6.91 (t, J_{PH} = 15.0 Hz, 1H, Ru-H);
¹⁹F-NMR (188 MHz, CD₂Cl₂, 28°C): δ = -152.6 (s);
³¹P-NMR (162.0 MHz, CD₂Cl₂, 28°C): δ = 55,5 (s, PCy₃);
¹³C-NMR (100.6 MHz, CD₂Cl₂, 28°C): δ = 84,6 (s, OCH₂CH₃), 41,4 (s, Ru≡C-Me), 35.7 (vt, N = 22.1 Hz, Cₐ von PCy₃), 31.2 und 30.4 (je s, PCy₃), 27.6 und 27.3 je vt, N = 10.2 Hz, PCy₃), 26.2 (s, PCy₃), 12.7 (s, OCH₂CH₃); das Carbin-CAtom wurde nicht detektiert.

Durch Umsetzung von 2 mit H₂O entsteht durch Ligandenaustausch [RuClH(≡C-Me)(OH₂)(PCy₃)₂][BF₄]:
¹H-NMR (400.1 MHz, CD₂Cl₂, -60°C): δ = 2.49-2.37, 1.94-1.09 (je m, 71 H, CH₃, H₂O, PCy₃) -6.48 (t breit, 1H, Ru-H);
¹⁹F-NMR (376.4 MHz, CD₂Cl₂, -60°C): δ = 151.4 (s);
³¹P-NMR (162.0 MHz, CD₂Cl₂, -60°C): δ = 56.2 (s, PCy₃);
¹³C-NMR (100.6 MHz, CD₂Cl₂, -60°C): δ = 313.9 (m breit, Ru≡C-Me), 40.8 (s, Ru≡C-Me), 34.1 (vt, N = 22.0 Hz, Cₐ von PCy₃), 30.6, 29.0, 26.8, 26.5 und 25.4 (je s, PCy₃).

### Beispiel 5: Progressive Umsetzung von RuHCl(=C=CH₂)(PCy₃)₂ mit CF₃SO₃H und HBF₄.

Zu der braunen Lösung von 56 mg (0.08 mmol) RuHCl(=C=CH₂)(PCy₃)₂ aus Beispiel 2 in 2 ml THF werden bei -78°C 6.8 µl (0.08 mmol) CF₃SO₃H zugetropft. Nach 10 min Rühren bei -78°C werden 2 ml einer 1.6 M etherischen HBF₄-Lösung (3.20 mmol) zugegeben. Man erhält eine gelbbraune Lösung 3.

### Beispiel 6: Ringöffnende Metathesepolymerisation (ROMP) von Cycloocten mit Mischung 3.

4 ml (30.7 mmol) Cycloocten werden bei Raumtemperatur mit einigen Tropfen der Mischung 3 versetzt. Nach 2 min ist die Lösung hochviskos. Nach geeigneter Aufarbeitung werden 3.5 g (99%) Polyoctenamer isoliert. Bei Einsatz der Katalysatoren 1 und 2 werden vergleichbare Resultate erhalten.

### Beispiel 7: Selektive ringöffnende Metathese (ROM) von Cyclopenten mit Acrylsäuremethylester

Eine Mischung aus 50 ml (0.56 mol) Acrylsäuremethylester und 4 ml (0.05 mmol) Cyclopenten wird bei Raumtemperatur mit einigen Tropfen der Mischung 3 versetzt und 2 h gerührt. Anschließend werden überschüssiges Substrat und Lösungsmittel bei Normaldruck abdestilliert, der verbleibende Rückstand mit 10 ml Pentaan versetzt und über Aluminiumoxid (neutral, Aktivitätsstufe III, Füllhöhe 2 cm) unter Zugabe von 60 ml Et₂O als Lösungsmittel filtriert, um Metallreste zu entfernen. Nach Abdestillieren des Lösungsmittels verbleiben 2.5 g einer farblosen Flüssigkeit. Diese enthält die ersten Glieder der homologen Reihe langkettiger und mehrfach ungesättigter Ester C₇H₁₁CO₂Me, C₁₂H₁₉CO₂Me und C₁₇H₂₇CO₂Me in Selektivitäten von 50, 40 und 10%.
Beim Einsatz der Katalysatoren 1 und 2 werden vergleichbare Resultate erhalten.

## Patentansprüche

1. Kationische Rutheniumkomplexe der allgemeinen Formeln A oder B oder diese enthaltende Gemische 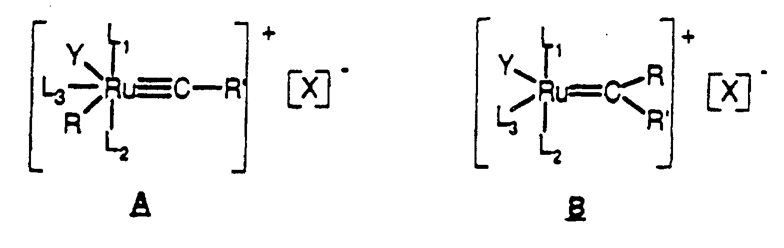 wobei B durch einen weiteren Liganden L₄ stabilisiert sein kann und
X ein nicht- oder schwach an das Metallzentrum koordinierendes Anion,
Y einen ein- oder mehrzähnigen anionischen Liganden,
R und R' jeweils unabhängig voneinander Wasserstoff oder einen gegebenenfalls substituierten, C₁₋₂₀-Alkyl-, C₆₋₂₀-Aryl- oder C₇₋₂₀-Alkylarylrest und
L₁, L₂, L₃ und L₄ unabhängig voneinander neutrale Elektronendonor-Liganden bedeuten.

2. Rutheniumkomplexe nach Anspruch 1, **dadurch gekennzeichnet, daß** die neutralen Elektronendonor-Liganden Phosphane, Arsane, Stibane mit mindestens zwei sterisch anspruchsvollen Resten, Amine, Pyridine, π-koordinierte Olefine oder Lösemittelmoleküle sind.

3. Verfahren zur Herstellung von kationischen Rutheniumkomplexen gemäß Anspruch 1 oder 2 durch Umsetzung eines neutralen Vinylidenkomplexes der allgemeinen Formel RuY(H)(=C=CHR)L₁L₂ mit R⁺X⁻, wobei X⁻ ein nicht- oder nur schwach an das Metallzentrum koordiniertes Anion oder Komplexanion und R⁺H⁺, Alkyl⁺, Aryl⁺, Alkylaryl⁺ oder ein Komplexkation des Typs LH⁺ darstellen.

4. Verfahren zur Herstellung von kationischen Rutheniumkomplexen gemäß Anspruch 1 oder 2 durch Umsetzung von neutralen Alkylidenkomplexen des Typs RuYY'(=CHR)L₁L₂, wobei Y = Y' sein kann mit R⁺X⁻, wobei X⁻ ein nicht- oder schwach an das Metallzentrum koordinierendes Anion oder Komplexanion, R⁺ H⁺, Alkyl⁺, Aryl⁺, Alkylaryl⁺ oder ein komplexes Kation des Typs LH⁺ bedeuten.

5. Verfahren zur Herstellung von kationischen Rutheniumkomplexen gemäß Anspruch 1 oder 2 durch Umsetzung von Verbindungen des Typs RuYY'(=CHR)L₁L₂ mit anionenabstrahierenden Metallsalzen M⁺X⁻ oder Lewissäuren in Gegenwart eines Liganden L₃, wobei X⁻ ein nicht- oder schwach koordinierendes Anion darstellt und die anionischen Liganden Y und Y' gleich oder voneinander verschieden sein können.

6. Verfahren nach Anspruch 3 oder 4, **dadurch gekennzeichnet, daß** R⁺X⁻ eine saure Verbindung H⁺X⁻ oder LH⁺X⁻ darstellt, wobei X⁻ ein nicht- oder schwach koordinierendes Anion oder Komplexanion darstellt und L ein Zweielektronendonor-Ligand ist.

7. Verfahren nach Anspruch 6, **dadurch gekennzeichnet, daß** R⁺X⁻ CF₃SO₃H, F₃CCO₂H, HClO₄, HBF₄, HPF₆, HSbF₆ oder [PhNMe₂H] [B(C₆F₅)₄] bedeutet.

8. Verfahren nach Anspruch 5, **dadurch gekennzeichnet, daß** als anionen- abstrahierende Metallsalze M⁺X⁻ solche Salze eingesetzt werden, bei denen M⁺ mit den zu abstrahierenden Anionen ein schwerlösliches neues Metallsalz bildet und X⁻ ein nicht- oder schwach koordinierendes Anion darstellt.

9. Verfahren nach Anspruch 8, **dadurch gekennzeichnet, daß** als M⁺X⁻ AgBF₄, AgB(C₅F₅)₄, AgPF₆ oder AgSbF₆ eingesetzt wird.

10. Verwendung von kationischen Rutheniumkomplexen gemäß Anspruch 1 oder 2 als Katalysatoren für Metathesereaktionen.

## Claims

1. A cationic ruthenium complex of the formula A or B or a mixture in which it is present 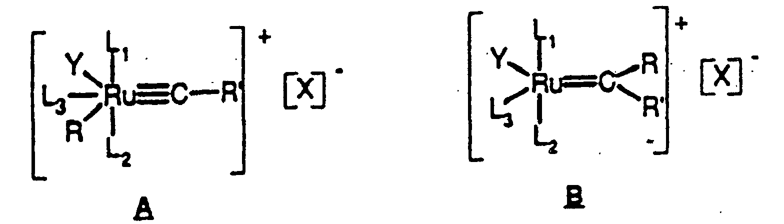 where B may be stabilized by a further ligand L₄ and
X is an anion which does not coordinate or coordinates only weakly to the metal center,
Y is a monodentate or multidentate anionic ligand,
R and R' are each, independently of one another, hydrogen or a substituted or unsubstituted C₁-C₂₀-alkyl, C₆-C₂₀-aryl or C₇-C₂₀-alkylaryl radical and
L₁, L₂, L₃ and L₄ are, independently of one another, uncharged electron donor ligands.

2. A ruthenium complex as claimed in claim 1, wherein the uncharged electron donor ligands are phosphines, arsines, stibines each bearing at least two bulky radicals, amines, pyridines, π-coordinated olefins or solvent molecules.

3. A process for preparing cationic ruthenium complexes as claimed in claim 1 or 2 by reacting an uncharged vinylidene complex of the formula RuY(H)(=C=CHR)L₁L₂ with R⁺X⁻, where X⁻ is an anion or complex anion which does not coordinate or coordinates only weakly to the metal center and R⁺ is H⁺; alkyl⁺, aryl⁺, alkylaryl⁺ or a complex cation of the type LH⁺.

4. A process for preparing cationic ruthenium complexes as claimed in claim 1 or 2, by reacting uncharged alkylidene complexes of the type RuYY'(=CHR)L₁L₂, where Y can be the same as Y', with R⁺X⁻, where X⁻ is an anion or complex anion which does not coordinate or coordinates weakly to the metal center and R⁺ is H⁺, alkyl⁺, aryl⁺, alkylaryl⁺ or a complex cation of the type LH⁺.

5. A process for preparing cationic ruthenium complexes as claimed in claim 1 or 2 by reacting compounds of the type RuYY'(=CHR)L₁L₂ with anion-abstracting metal salts M⁺X⁻ or Lewis acids in the presence of a ligand L₃, where X⁻ is a non-coordinating or weakly coordinating anion and the anionic ligands Y and Y' can be identical or different.

6. A process as claimed in claim 3 or 4, wherein R⁺X⁻ is an acid compound H⁺X⁻ or LH⁺X⁻, where X⁻ is a non-coordinating or weakly coordinating anion or complex anion and L is a two-electron donor ligand.

7. A process as claimed in claim 6, wherein R⁺X⁻ is CF₃SO₃H, F₃CCO₂H, HClO₄, HBF₄, HPF₆, HSbF₆ or [PhNMe₂H] [B(C₆F₅)₄].

8. A process as claimed in claim 5, wherein the anion-abstracting metal salts M⁺X⁻ used are salts in which M⁺ forms a sparingly soluble, new metal salt with the anions to be abstracted and X⁻ is a non-coordinating or weakly coordinating anion.

9. A process as claimed in claim 8, wherein M⁺X⁻ is AgBF₄, AgB(C₅F₅)₄, AgPF₆ or AgSbF₆.

10. The use of a cationic ruthenium complex as claimed in claim 1 or 2 as a catalyst for metathesis reactions.

## Revendications

1. Complexes cationiques de ruthénium de la formule générale A ou B ou mélanges contenant ces derniers 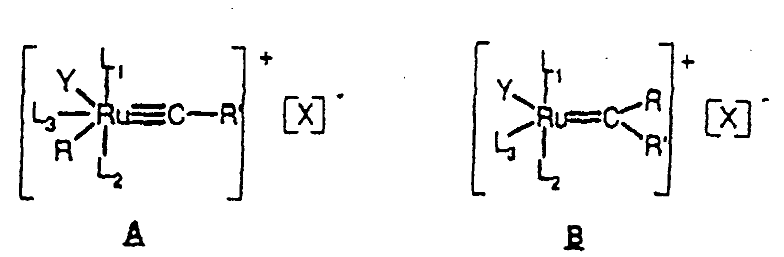 dans lequel cas, B peut être stabilisé par un autre ligand L4
X représente un anion pas ou peu coordinant au noyau métallique,
Y représente des ligands anioniques monodentés ou polydentés,
R et R', indépendamment l'un de l'autre sont de l'hydrogène ou C₁₋₂₀₋alkyle, C₆₋₂₀₋aryle ou un reste de C₇₋₂₀₋alkylaryle, substitué le cas échéant,
L₁, L₂, L₃ et L₄, indépendamment l'un de l'autre sont des ligands neutres donneurs d'électrons

2. Complexes de ruthénium selon la revendication 1, **caractérisés en ce que** les ligands neutres donneurs d'électrons, sont des phosphanes, des arsanes, des stibanes avec au moins deux restes à haute exigence stérique, des amines, des pyridines, des oléfines coordonnées π ou des molécules de solvant.

3. Méthode de préparation de complexes cationiques de ruthénium selon l'une quelconque des revendications 1 ou 2, par transformation d'un complexe vinylidène neutre de la formule générale RuY(H)(=C=CHR)L₁L₂, avec R⁺X₋, dans lequel cas X⁻ est un anion ou un anion complexe peu ou faiblement coordonné au noyau métallique et R⁺ H⁺, correspondent à de l'alkyle⁺, de l'aryle⁺, de l'alkylaryle⁺ ou à un cation complexe du type LH⁺.

4. Méthode de préparation de complexes cationiques de ruthénium selon l'une quelconque des revendications 1 ou 2, par transformation d'un complexe d'alkylidènes neutres du type RuYY1(=CHR)L₁L₂, dans lequel cas Y peut être = Y', avec R⁺X⁻, dans lequel cas X⁻ est un anion ou un anion complexe peu ou faiblement coordinant au noyau métallique et R⁺ H⁺, correspondent à de l'alkyle+, de l'aryle⁺, de l'alkylaryle⁺ ou à un cation complexe du type LH⁺.

5. Méthode de préparation de complexes cationiques de ruthénium selon l'une quelconque des revendications 1 ou 2, par transformation de composé du type Ru YY'(=CHR)L₁L₂, avec des sels métalliques abstracteurs d'anions M⁺X⁻ ou des acides de Lewis, en présence d'un ligand L₃, dans lequel cas X⁻ est un anion non ou faiblement coordinant et les ligands anioniques Y et Y' peuvent être identiques ou différents l'un de l'autre.

6. Méthode selon l'une quelconque des revendications 3 ou 4, **caractérisée en ce que** R⁺X⁻ représentent un composé acide H⁺X⁻ ou LH⁺X⁻, dans lequel cas X⁻ représente un anion complexe peu ou faiblement coordinant et L est un ligand donneur de deux électrons.

7. Méthode selon la revendication 6, **caractérisée en ce que** R⁺X⁻ signifient CF₃SO₃H, F₃CCO₂H, HClO₄, HBF₄, HPF₆, HSbF₆ ou [PhNMe₂H] [B(C₆F₅)₄].

8. Méthode selon la revendication 5, **caractérisée en ce que** pour les sels métalliques abstracteurs d'anion M⁺X⁻, on peut utiliser des sels dans lesquels M⁺ forme avec les anions à abstraire un nouveau sel métallique difficilement soluble et X⁻ est un anion non ou faiblement coordinant.

9. Méthode selon la revendication 8, **caractérisé en ce que**, pour M⁺, X⁻, on utilise AgBF₄, AgB(C₅F₅)₄, AgPF₆ ou AgSbF₆.

10. Utilisation de complexes cationiques de ruthénium selon l'une quelconque des revendications 1 ou 2 en tant que catalyseurs de réactions de métathèse.
